# EUROPÄISCHE PATENTANMELDUNG

(11) **EP 3 165 217 A1**
(43) Veröffentlichungstag der Anmeldung: **10.05.2017**
(21) Anmeldenummer: 16173179.9
(22) Anmeldetag: 06.06.2016
(51) Int. Cl.: A61K 8/46, A61K 8/86, A61Q 19/10, A61K 8/92, A61K 8/97

(54) **KÖRPERREINIGUNGSMITTEL**

(30) Priorität: 05.11.2015 DE 202015105918 U
(71) Anmelder: Schmidt-Bartl, Heidrun, 11140 Conil de la Frontera (Cadiz) (ES)
(72) Erfinder: Schmidt-Bartl, Heidrun, 11140 Conil de la Frontera (Cadiz) (ES)
(74) Vertreter: Limbeck, Achim

(57) **Zusammenfassung**

Die vorliegende Erfindung betrifft ein Körperreinigungsmittel auf wässriger Basis, welches erfindungsgemäß dadurch gekennzeichnet ist, dass mindestens etwa 50 bis etwa 99 Gew. -% keimfreies Quellwasser enthalten ist und darüber hinaus mindestens 1 bis etwa 50 Gew. -% eines Detergens enthalten ist, das natürliche Inhaltsstoffe und/oder andere natürliche Tenside enthält und für Reinigungsprozesse bestimmt ist.

## Beschreibung

### Technisches Gebiet

Die vorliegende Erfindung betrifft ein Körperreinigungsmittel auf wässriger Basis. Das Körperreinigungsmittel ist vorzugsweise aber nicht ausschließlich mit einer mechanischen Sprühvorrichtung versprühbar und soll insbesondere zur Reinigung und Hygiene pflegebedürftiger Personen in der Alten- und Behindertenpflege sowie in der stationären und häuslichen Krankenpflege verwendet werden.

### Stand der Technik

Herkömmliche Körperreinigungsmittel enthalten üblicherweise neben den für die Reinigungswirkung ursächlichen Tensiden, welche bewirken, dass zwei eigentlich nicht miteinander mischbare Flüssigkeiten, wie bspw. Öl und Wasser, fein vermengt werden können, zusätzlich spezielle hautpflegende Zusätze, die gewährleisten sollen, dass die Anwendung dieser Körperreinigungsmittel ein angenehmes und gepflegtes Hautgefühl erzeugt.

Nachteilig hierbei ist, dass bekannte Körperreinigungsmittel in der Regel sowohl durch deren Inhaltsstoffe als auch ihre Konsistenz arbeits- und materialaufwendig in ihrer Anwendung sind und zumeist eine unvollständige Reinigung und Pflege bewirken, wobei hinzu kommt, dass bekannte Mittel insbesondere bei Personen höheren Alters, welche hier vorzugsweise angesprochen werden, zu Hautproblemen führen, da diese eine deutlich empfindlichere Haut aufweisen als jüngere Anwender.

### Darstellung der Erfindung

Der vorliegenden Erfindung liegt die Aufgabe zu Grunde, ein Körperreinigungsmittel zu schaffen, welches die vorgenannten Nachteile ausräumt und welches sich speziell für die Reinigung und Hygiene pflegebedürftiger Personen eignet.

Erfindungsgemäß wird die voranstehende Aufgabe gemäß dem Oberbegriff des Anspruchs 1 in Verbindung mit den kennzeichnenden Merkmalen gelöst. Vorteilhafte Ausgestaltungen und Weiterbildungen des erfindungsgemäßen Körperreinigungsmittels sind in den abhängigen Unteransprüchen angegeben.

Erfindungsgemäß ist ein Körperreinigungsmittel der eingangs genannten Art dadurch gekennzeichnet, dass mindestens etwa 50 bis etwa 99 Gew. -% keimfreies Quellwasser enthalten ist und darüber hinaus mindestens 1 bis etwa 50 Gew.-% eines Detergens enthalten ist, das natürliche Inhaltsstoffe und/oder andere natürliche Tenside enthält und welches für Reinigungsprozesse bestimmt ist.

Als Quellwasser sei nachstehend Wasser verstanden, das aus natürlichen unterirdischen und schadstoffgeschützten Reservoiren stammt und direkt am Ort der Quelle abgefüllt wird. Der Vorteil der Verwendung von Quellwasser besteht darin, dass sich dieses Wasser aus Oberflächenwasser speist, das auf seinem Weg in die Tiefe verschiedene Gesteinsschichten passiert, damit einem natürlichen Filtrationsprozess unterliegt und somit ebenso wie das Wasser aus den tieferen Bodenschichten selbst keimfrei ist. Gleichzeitig können sich Mineralsalze und Spurenelemente aus dem Gestein lösen und dem Wasser darüber hinaus eine physiologische Wirkung verleihen.

Das verwendete Quellwasser ist erfindungsgemäß natürlich rein und unterliegt keinem chemischen Filtrierungsprozess, wird also nicht weiter aufbereitet. Grundsätzlich ist es allerdings denkbar, verschiedene Verfahren zur Abtrennen von Eisen, Mangan, Schwefelverbindungen sowie Arsen oder Ozonierung durchzuführen, solange der ursprüngliche Charakter des in dem erfindungsgemäßen Körperreinigungsmittel enthaltenen Quellwassers durch diese Prozesse nicht verändert wird.

Als natürliche Inhaltstoffe für ein Detergen kommen grundsätzlich eine Vielzahl von Pflanzen in Betracht wie bspw. Algen, welche sehr vitamin- und mineralstoffreich sind und als straffend und feuchtigkeitsspendend gelten.

Darüber hinaus eignet sich als natürliches Detergen auch Aloe Vera. Aloe Vera ist ein wirksamer Feuchtigkeitsspender und hat nebenbei noch einen leichten frischen Duft. Ferner eignen sich Avocado, deren natürliche Inhaltsstoffe reich an Vitamin A und E sind und Wildrosenöl, welches sich gut auf (trockener) Haut verteilen lässt und schnell einzieht.

Als Tenside natürlichen Ursprungs eignen sich bspw. Seifen, die aus natürlichen Rohstoffen (nachwachsenden Rohstoffen, zum Beispiel aus pflanzlichen oder tierischen Fetten) durch Verseifung hergestellt werden.

Auf Basis von Fetten und den daraus gewonnenen Fettalkoholen können außerdem Tenside wie Fettalkoholpolyglycolether (FAE), Fettalkoholsulfate (FAS), Fettalkoholethersulfate (FAES, sulfierte Fettalkoholpolyglycolether) und Methylestersulfonate (MES, sulfonierte Fettsäuremethylester) Verwendung finden.

In einer besonders vorteilhaften Ausführungsform ist dem Körperreinigungsmittel ein Pflanzenöl in Form von Olivenöl oder kaltgepresstem Olivenöl beigemischt.

Das erfindungsgemäße Körperreinigungsmittel kann zur Pflege von Gesicht, Händen und Körper verwendet werden und wird in einer vorteilhaften Ausführungsform durch Aufsprühen auf die Haut aufgetragen. Anschließend werden die besprühten Körperpartien mittels Papiertüchern gesäubert.

Die Verwendung des erfindungsgemäßen Körperreinigungsmittel dient neben seiner reinigenden Wirkung als Dekubitus-Prophylaxe durch gründliche rückstandsfreie und pflegende Reinigung aller Körperregionen, ohne Zusatz von Konservierungsmitteln, Aromastoffen und Deodorantien.

Ein weiterer zu beobachtender Effekt des erfindungsgemäßen Körperreinigungsmittels besteht darin, dass dieser aufgrund seiner Inhaltsstoffe bereits beim Besprühen der Intimregion eine Geruchsbelästigung verhindert.

Das erfindungsgemäße Körperreinigungsmittel beschränkt sich in seiner Ausführung nicht auf die vorstehend angegebenen bevorzugten Ausführungsformen. Vielmehr sind eine Vielzahl von Ausgestaltungsvariationen denkbar, welche von der dargestellten Lösung auch bei grundsätzlich anders gearteter Ausführung Gebrauch machen.

## Patentansprüche

1. Körperreinigungsmittel auf wässriger Basis,
**dadurch gekennzeichnet, dass**
mindestens etwa 50 bis etwa 99 Gew. -% keimfreies Quellwasser enthalten ist und darüber hinaus mindestens 1 bis etwa 50 Gew. -% eines Detergens enthalten ist, das natürliche Inhaltsstoffe und/oder andere natürliche Tenside enthält und für Reinigungsprozesse bestimmt ist.

2. Körperreinigungsmittel nach Anspruch 1,
**dadurch gekennzeichnet, dass**
als natürliche Inhaltstoffe für ein Detergen Pflanzen dienen, welche sehr vitamin- und mineralstoffreich sind und als straffend und feuchtigkeitsspendend gelten.

3. Körperreinigungsmittel nach den Ansprüchen 1 und 2,
**dadurch gekennzeichnet, dass**
als natürliche Inhaltstoffe für ein Detergen Algen, Aloe Vera und/oder Avocado enthalten sind.

4. Körperreinigungsmittel nach den vorangegangenen Ansprüchen,
**dadurch gekennzeichnet, dass**
als Tenside natürlichen Ursprungs Seifen enthalten sind, die aus natürlichen Rohstoffen durch Verseifung hergestellt sind.

5. Körperreinigungsmittel nach den vorangegangenen Ansprüchen,
**dadurch gekennzeichnet, dass**
verschiedene Verfahren zur Abtrennen von Eisen, Mangan, Schwefelverbindungen sowie Arsen oder Ozonierung durchgeführt werden, solange der ursprüngliche Charakter des in dem erfindungsgemäßen Körperreinigungsmittel enthaltenen Quellwassers durch diese Prozesse nicht verändert wird.

6. Körperreinigungsmittel nach den vorangegangenen Ansprüchen,
**dadurch gekennzeichnet, dass**
dem Körperreinigungsmittel ein Pflanzenöl in Form von Olivenöl oder kaltgepresstem Olivenöl beigemischt ist.
